# EUROPEAN PATENT APPLICATION

(11) **EP 4 591 825 A1**
(43) Date of publication of application: **30.07.2025**
(21) Application number: 23867970.8
(22) Date of filing: 28.08.2023
(51) Int. Cl.: A61C 13/083, A61C 5/77, C04B 35/488, C04B 41/85

(54) **METHOD FOR COLORING DENTAL CERAMIC, AND COLORING SOLUTION FOR DENTAL CERAMIC**

(30) Priority: 22.09.2022 JP 2022151021
(71) Applicant: GC Corporation, Sunto-gun, Shizuoka 410-1307 (JP)
(72) Inventor: NAGAOKA, Kento, Tokyo 174-8585 (JP); ONODERA, Mizuho, Tokyo 174-8585 (JP); YAMAMOTO, Koji, Tokyo 174-8585 (JP); HOKII, Yusuke, Tokyo 174-8585 (JP); AKIYAMA, Shigenori, Tokyo 174-8585 (JP)
(74) Representative: Müller-Boré & Partner Patentanwälte PartG mbB
(86) International application number: PCT/JP2023/031031
(87) International publication number: WO 2024/062846

(57) **Abstract**

A method for coloring a dental ceramic (10) includes a step of contacting a coloring liquid (30) with the dental ceramic (10). The coloring liquid (30) contains at least two types of metal ions in which a difference between standard oxidation-reduction potentials is 0.5 or more and 4.0 or less.

## Description

### TECHNICAL FIELD

The present invention relates to a method for a coloring dental ceramic, and a coloring liquid for a dental ceramic.

### BACKGROUND ART

Patent literature (PTL) 1 discloses a technology of manufacturing a dental prosthesis having a desired shape by grinding and cutting a gradation block formed of ceramics, such as zirconia or the like, using CAD/CAM (computer-aided design/computer-aided manufacturing).

PTL 2 discloses a method of manufacturing a dental calcined body or a dental fired body colored for manufacturing a dental restoration using CAD/CAM. The disclosed method includes sequentially dropping and permeating a plurality of coloring liquids of different colors to a calcined body obtained through calcination of a product obtained by molding ceramics, such as zirconia or the like.

### CITATION LIST

### PATENT LITERATURE

PTL 1: International Publication No. 2009/154301
PTL 2: Japanese Patent No. 6093900

### SUMMARY OF THE INVENTION

### TECHNICAL PROBLEM

However, for existing dental ceramics, there is a need to provide a plurality of coloring liquids and perform a coloring step over and over again. This requires much time and effort.

An object of the present invention is to provide a method for coloring a dental ceramic in which a gradation is formed inside the dental ceramic in a single coloring step using a single coloring liquid.

### SOLUTION TO THE PROBLEM

A method for coloring a dental ceramic according to an embodiment of the present invention is a dental ceramic coloring method including a step of contacting a coloring liquid with the dental ceramic. The coloring liquid contains at least two types of metal ions in which a difference between standard oxidation-reduction potentials is 0.5 or more and 4.0 or less.

### ADVANTAGEOUS EFFECTS OF THE INVENTION

According to an embodiment of the present invention, it is possible to provide a method for coloring a dental ceramic in which a gradation is formed inside the dental ceramic in a single coloring step using a single coloring liquid.

### BRIEF DESCRIPTION OF THE DRAWINGS

[FIG. 1] FIG. 1 is a view illustrating an example of a dental ceramic.
[FIG. 2] FIG. 2 is a view illustrating an example of a dental ceramic coloring method.
[FIG. 3] FIG. 3 is a view illustrating an experimental example of the dental ceramic coloring method.
[FIG. 4] FIG. 4 is a chart illustrating results of Experimental Examples 1 to 10 of dental ceramics.
[FIG. 5] FIG. 5 is a chart illustrating results of Experimental Examples 11 to 14 of dental ceramics.
[FIG. 6] FIG. 6 is a chart illustrating results of Experimental Examples 15 to 18 of dental ceramics.
[FIG. 7] FIG. 7 is a chart illustrating results of Experimental Examples 19 to 22 of dental ceramics.
[FIG. 8] FIG. 8 is a chart illustrating results of Experimental Examples 23 to 26 of dental ceramics.
[FIG. 9] FIG. 9 is a chart illustrating results of Experimental Examples 27 and 28 of dental ceramics.

### DETAILED DESCRIPTION OF THE INVENTION

Hereinafter, embodiments of the present invention will be described in detail.

### <Dental Ceramic Coloring Method>

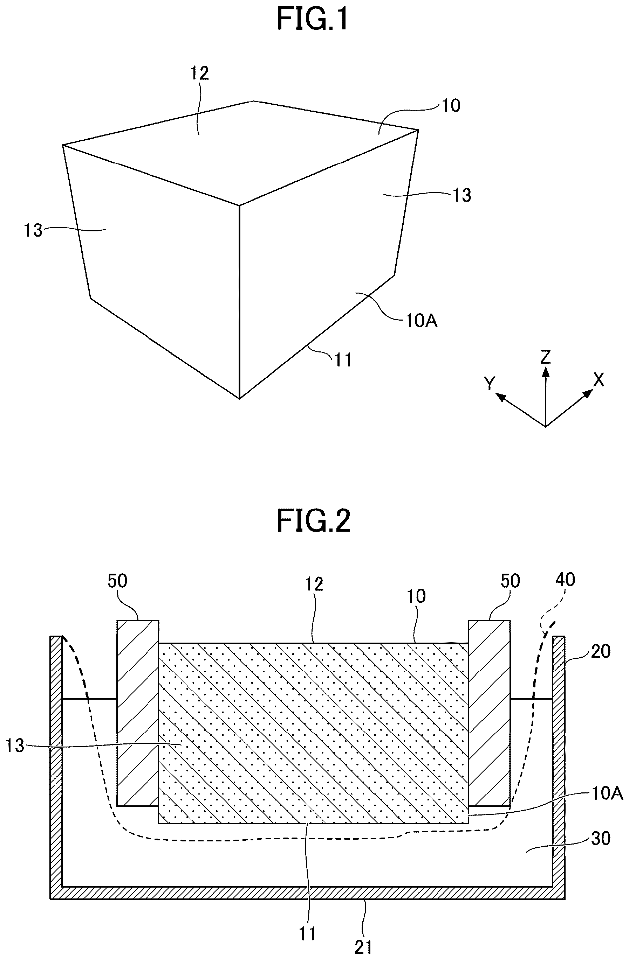

FIG. 1 is a view illustrating an example of a dental ceramic, and FIG. 2 is a view illustrating an example of a dental ceramic coloring method. The dental ceramic coloring method is a method of coloring a dental ceramic with a coloring liquid.

No particular limitation is imposed on the form of the dental ceramic. From the viewpoint of increasing coloring compatibility of a dental ceramic, the dental ceramic is preferably a molded body (molded ceramic body) or a calcined body (calcined ceramic body).

The molded body is formed by molding ceramics. A method of manufacturing the molded body is any method, and may be, for example, a method of charging ceramic powder into a mold, followed by compression molding; a method of charging a ceramic slurry into a mold, followed by hardening (slip casting, gel casting, or the like); or a lamination shaping method (additive manufacturing, 3D printing, or the like).

The calcined body is a product (calcined product) obtained by calcinating a molded body (e.g., heating a molded body at 600°C to 1,500°C). This calcined body has a state in which ceramic powder particles are temporarily bonded together, and is in a state in which a coloring liquid described below can permeate between the particles.

No particular limitation is imposed on the shape of a dental ceramic. The dental ceramic may be a block or a shaped product.

The block is a molded body or a calcined body before being processed into a shaped product, such as a dental prosthesis or the like. No particular limitation is imposed on the shape of the block, and the shape of the block may be, for example, a cubic shape, a rectangular parallelepiped shape, a cylindrical shape, or the like. The present embodiment will be described using, as a dental ceramic 10, a block having a rectangular parallelepiped shape (quadrangular prism shape) as illustrated in FIG. **1****.** However, the dental ceramic is not limited to this.

The shaped product has a predetermined shape of, for example, a dental prosthesis or the like. The shaped product is obtained by cutting a molded body or a calcined body using CAD/CAM (computer-aided design/computer-aided manufacturing) or the like.

Also, the shaped product may be obtained as a molded product having a predetermined shape, for example, by a lamination shaping method (e.g. additive manufacturing, 3D printing, or the like). That is, the molded body obtained by the lamination shaping method may be directly used as a shaped product, such as a dental prosthesis or the like.

The obtained shaped product may be fired (e.g., heated at 900°C to 2,100°C), if necessary.

Examples of the ceramics include, for example, glass containing silicate, borosilicate, aluminosilicate, phosphate, fluorophosphate, or the like, and glass ceramics thereof; and zirconia, alumina, feldspar, leucite, sanidine, spinel, mica, apatite, zircon, and mixtures thereof.

The dental ceramic coloring method according to the present embodiment includes a coloring step. In the coloring step, a dental ceramic is contacted by a coloring liquid. No particular limitation is imposed on a manner in which a dental ceramic is contacted by a coloring liquid. Examples of the manner include: applying, spraying, or dropping the coloring liquid onto the dental ceramic; dipping the entirety of or a part of the dental ceramic in the coloring liquid; and the like.

In the present embodiment, the coloring step preferably includes a step of dipping one end 10A of the dental ceramic 10 in a coloring liquid 30 (coloring step). Thus, the coloring liquid 30 can permeate through the dental ceramic 10 from a bottom surface 11 toward a top surface 12 (FIG. 2).

In the coloring step, as illustrated in FIG. 2, the coloring liquid 30 is charged to a container 20 that is cylindrical. Then, the dental ceramic 10 having a rectangular parallelepiped shape is placed in the container 20 in a state in which the bottom surface 11 (the one end 10A) of the dental ceramic 10 is dipped in the coloring liquid 30. The dental ceramic 10 is left to stand still for 1 hour to 20 hours (18 hours in the present embodiment) in a state of being dipped in the coloring liquid 30.

The arrangement for placing the dental ceramic 10 in the container 20 includes, for example, spreading out the mesh 40 within the container, and placing the dental ceramic 10 on the mesh 40 such that the ceramic 10 does not come into contact with a bottom surface 21 of the container 20 (FIG. 2).

Also, a range of the dental ceramic 10 dipped in the coloring liquid 30 is, for example, a range in which a side surface (a periphery 13) of the dental ceramic 10 covered by a covering material 50 is exposed by about 1/10 to about 1/4 in a height direction (Z direction) from the bottom end (the bottom surface 11) of the dental ceramic 10 (FIG. 2). The side surface (the periphery 13) of the dental ceramic 10 is four rectangular surfaces when the dental ceramic 10 has a quadrangular prism shape, and is one curved surface when the dental ceramic 10 has a cylindrical shape.

No particular limitation is imposed on the material of the covering material 50. Examples of the material include silicone rubber, resins, wax, and the like. The covering material 50 is preferably a flexible material from the viewpoint of enhancing adhesiveness with the dental ceramic 10 in covering the side surface 13 of the dental ceramic 10. Further, the flexible material is preferably silicone rubber or the like from the viewpoint of chemical resistance to the coloring liquid. In the present embodiment, for example, a silicone impression material can be used as the covering material 50.

The covering material 50 covers the entire periphery of the dental ceramic 10 except for the side surface 13 of the dental ceramic 10 that is dipped in the coloring liquid 30. In the present embodiment, in a state in which the bottom surface 11 and the top surface 12 of the dental ceramic 10 are exposed (left bare), the side surface 13 of the dental ceramic 10 is covered by the covering material 50.

The covering material 50 has a height that projects upward from the top surface 12 of the dental ceramic 10. Thus, in a state in which the dental ceramic 10 is placed in the container 20, it is possible to prevent permeation of the coloring liquid 30 from the top surface 12 of the dental ceramic 10.

No particular limitation is imposed on the shape of the covering material 50. For example, when the dental ceramic 10 has a quadrangular prism shape, the covering material 50 has a hollow quadrangular prism shape that is open at both ends. When the dental ceramic 10 has a cylindrical shape, the covering material 50 has a hollow cylindrical shape that is open at both ends.

The coloring liquid 30 contains at least two types of metal ions. The difference between standard oxidation-reduction potentials of these two types of metal ions is 0.5 or more and 4.0 or less, preferably 0.7 to 3.5, and more preferably 0.72 to 3.2.

In the present specification, the oxidation-reduction potential refers to an electrode potential (standard electrode potential) generated by electron transfer in a system (reaction system) in which an oxidation-reduction reaction occurs in an aqueous solution or the like. The difference between the standard oxidation-reduction potentials refers to an absolute value of the difference between the standard oxidation-reduction potentials of the two types of the metal ions contained in the coloring liquid 30.

Specifically, the coloring liquid 30 contains a pigment and a solvent.

No particular limitation is imposed on the solvent contained in the coloring liquid 30, but a polar solvent, such as water, alcohol, or the like, is used. In the present embodiment, water is used as the solvent.

The solvent may optionally contain a water-soluble polymer. As the water-soluble polymer, it is preferable to use, for example, polyalkylene glycol, such as polyethylene glycol, or a saccharide, such as sucrose, agarose, or the like. The coloring liquid of the present embodiment contains polyethylene glycol in water.

The content of the water-soluble polymer is, for example, 0.001% by mass or more and 20% by mass or less, preferably 2.0% by mass or more and 18.0% by mass or less, and more preferably 4.0% by mass or more and 15% by mass or less, with respect to 100% by mass of the coloring liquid.

A pigment contained in the coloring liquid 30 corresponds to at least two types of metal ions in which the difference between standard oxidation-reduction potentials is 0.5 or more and 4.0 or less.

One metal ion of these two types of metal ions (hereinafter may be referred to as metal ion A) is a metal ion having a standard oxidation-reduction potential of preferably -1.0 V or more and 2.0 V or less, more preferably -0.7 V or more and 1.5 V or less, and further preferably -0.5 V or more and 1.0 V or less.

No particular limitation is imposed on the metal ion A as long as the metal ion A forms a pigment. Examples of the metal ion A include Cr³⁺, Fe³⁺, Fe²⁺, Pd²⁺, Bi³⁺, Ge²⁺, Sn⁴⁺, Ni²⁺, Yb³⁺, Nb³⁺, Co²⁺, and the like. Also, the metal ion A may be a metal salt of, for example, a chloride, a sulfide, an acetate, a nitrate, or the like. Examples of the metal salt include CrCl₃, FeCl₃, PdCl₂, BiCl₃, GeCl₄, SnCl₂, NiCl₂, YbCl₃, NbCl₅, CoCl₂, and the like. These metal salts may be a hydrate.

The metal ion A may be used alone or in combination. Of these, the metal ion A is preferably Fe³⁺, Fe²⁺, Ni²⁺, Yb³⁺, Nb³⁺, or Co²⁺ due to the ability of these preferred metal ions to readily produce a hue or tinge that closely resembles a natural tooth color/structure.

No particular limitation is imposed on the content of the metal ion A. The metal ion A is contained as a metal salt in 100% by mass of the coloring liquid in an amount of preferably 0.0001% by mass or more and 5.0% by mass or less, more preferably 0.0005% by mass or more and 3.0% by mass or less, and further preferably 0.001% by mass or more and 2.0% by mass or less.

When the metal ion A is contained as a metal salt in 100% by mass of the coloring liquid in an amount of 0.0001% by mass or more and 5.0% by mass or less, an appropriate degree of hue or tinge corresponding to the metal ion A can be imparted to the dental ceramic 10.

Of the two types of metal ions, the other metal ion (hereinafter may be referred to as metal ion B) has a standard oxidation-reduction potential of preferably -3.0 V or more and -0.7 V or less, more preferably -2.5 V or more and -0.9 V or less, and further preferably -2.4 V or more and -1.0 V or less.

No particular limitation is imposed on the metal ion B as long as the metal ion B forms a pigment. Examples of the metal ion B include Mn²⁺, Ce³⁺, Nd³⁺, Pr³⁺, Sm³⁺, Eu³⁺, Tb³⁺, Dy³⁺, Ho³⁺, Er³⁺, Y³⁺, and the like.

The metal ion B may be a metal salt of, for example, a chloride, a sulfide, an acetate, a nitrate, or the like. Examples of the metal salt include MnCl₂, CeCl₃, NdCl₃, PrCl₃, SmCl₃, EuCl₃, TbCl₃, DyCl₃, HoCl₃, ErCl₃, YCl₃, and the like. These metal salts may be a hydrate.

The metal ion B may be used alone or in combination. Of these, the metal ion B is preferably Mn²⁺, Ce³⁺, Nd³⁺, Pr³⁺, Er³⁺, or Y³⁺ due to the ability of these preferred metal ions to readily produce a hue or tinge that closely resembles a natural tooth color/structure.

No particular limitation is imposed on the content of the metal ion B. The metal ion B is contained as a metal salt in 100% by mass of the coloring liquid in an amount of preferably 0.0001% by mass or more and 5.0% by mass or less, more preferably 0.0005% by mass or more and 3.0% by mass or less, and further preferably 0.001% by mass or more and 2.0% by mass or less.

When the metal ion B is contained as a metal salt in 100% by mass of the coloring liquid in an amount of 0.0001% by mass or more and 5.0% by mass or less, an appropriate degree of hue or tinge corresponding to the metal ion B can be imparted to the dental ceramic 10.

The coloring liquid 30 may further contain a chelating agent. The chelating agent forms complexes with some of the metal ions contained in the coloring liquid, and the metal ions forming the complexes (metal ion complexes) have large steric hindrance and a relatively low migration speed in the dental ceramic 10. Therefore, when the coloring liquid contains a chelating agent, it is possible to control the migration speed, in the dental ceramic 10, of the metal ions that tend to form complexes with the chelating agent.

No particular limitation is imposed on the chelating agent. Examples of the chelating agent include ethylenediamine-N,N,N',N'-tetraacetic acid (EDTA), diethylenetriamine-N,N,N',N'',N''-pentaacetic acid (DTPA), N-hydroxyethylethylenediamine-N,N',N'-triacetic acid (HEDTA), nitrilotriacetic acid (NTA), O,O'-bis(2-aminoethyl)ethyleneglycol-N,N,N',N'-tetraacetic acid (EGTA), trans-1,2-diaminocyclohexane-N,N,N',N'-tetraacetic acid (CyDTA), citric acid, tartaric acid, malic acid, oxalic acid, gluconic acid, tripolyphosphoric acid, 12-crown 4-ether, and salts thereof.

The chelating agent may be used alone or in combination. Of these, EDTA is preferable as the chelating agent because of readily forming complexes with Fe³⁺ and Er³⁺, which readily produce a hue or tinge of the tooth substance.

No particular limitation is imposed on the content of the chelating agent. The chelating agent is preferably contained in 100% by mass of the coloring liquid in an amount of 0.00001% by mass or more and 10.0% by mass or less. When the content of the chelating agent is less than 0.00001% by mass, the effect of the chelating agent is not readily obtained, whereas even if the chelating agent is contained in an amount of more than 10.0% by mass, the obtained effect is not appreciably changed.

The pH of the coloring liquid is preferably 8 or less, more preferably 5 or less, and further preferably 3 or less. When the pH of the coloring liquid is 8 or less (substantially neutral or acidic), some metal ions (e.g., Fe³⁺, Pr³⁺, and Er³⁺) tend to maintain or increase the ionic forms in the coloring liquid (tend to maintain or decrease the molecular forms in the coloring liquid). Therefore, when the pH of the coloring liquid is 8 or less, even if the concentration in the coloring liquid is low, the migration speed in the dental ceramic 10 increases, thereby stabilizing coloring.

The dental ceramic coloring method according to the present embodiment optionally includes a drying step and a firing step after the coloring step.

In the drying step, the dental ceramic 10 through which the coloring liquid 30 has permeated in the coloring step is taken out from the container 20, and dried at room temperature for 1 hour to 6 hours. Further, the dried dental ceramic 10 is cut out into a desired size. In the present embodiment, the dried dental ceramic 10 is cut (sliced) so as to have a thickness of about 1.5 mm.

In the firing step, the dental ceramic 10 cut out into a desired size in the drying step is fired at 1,000°C to 2,100°C (about 1,500°C in the present embodiment) for 30 minutes to 5 hours (2 hours in the present embodiment). The firing in this firing step corresponds to main firing (firing that is different from calcination).

In the present embodiment, as described above, the dental ceramic coloring method includes a step of contacting the dental ceramic with the coloring liquid containing at least two types of metal ions in which a difference between standard oxidation-reduction potentials is 0.5 or more and 4.0 or less (coloring step). Thus, it is possible to obtain a dental ceramic in which a gradation is formed inside the dental ceramic in a single coloring step using a single coloring liquid.

In the present embodiment, as described above, by dipping one end of the dental ceramic in the coloring liquid in the coloring step, it is possible to reliably obtain a dental ceramic in which a gradation is formed inside the dental ceramic in a single coloring step using a single coloring liquid.

Also, in the present embodiment, as described above, the standard oxidation-reduction potential of one of the two types of the metal ions (the metal ion A) is -1.0 V or more and 2.0 V or less, and thus metal ions having a relatively high standard oxidation-reduction potential (having an unstable ionic state) exist in the coloring liquid. As a result, the steric hindrance of the metal ion A increases, and thus the migration speed of the metal ion A in the dental ceramic 10 is relatively low. From this point of view, it is possible to achieve a good balance of coloring in the dental ceramic.

Also, in the present embodiment, as described above, the standard oxidation-reduction potential of the other of the two types of the metal ions (the metal ion B) is -3.0 V or more and 0.7 V or less, and thus metal ions having a relatively low standard oxidation-reduction potential (having a stable ionic state) exist in the coloring liquid. As a result, the steric hindrance of the metal ion B decreases, and thus the migration speed of the metal ion B in the dental ceramic 10 is relatively high. From this point of view, it is possible to achieve a good balance of coloring in the dental ceramic.

Also, in the present embodiment, as described above, the coloring liquid further contains a water-soluble polymer. Therefore, dispersibility of the metal ions in the coloring liquid is improved, and thus unevenness in coloring in the dental ceramic 10 can be suppressed.

Also, in the present embodiment, as described above, the coloring liquid further contains a chelating agent. Thus, the chelating agent forms complexes with some of the metal ions contained in the coloring liquid (metal ion complexes). Further, the metal ion complexes have a steric hindrance greater than the metal ions, which do not form complexes. Thus, the migration speed of the metal ion complexes in the dental ceramic 10 becomes lower. In the present embodiment, from this point of view, it is possible to control the migration speed, in the dental ceramic 10, of the metal ions that readily form complexes with the chelating agent.

Also, in the present embodiment, as described above, the pH of the coloring liquid is 8 or less. Thus, ionic forms tend to be maintained or increased (molecular forms tend to be maintained or decreased) in the coloring liquid. Therefore, even if the concentration in the coloring liquid is low, the migration speed in the dental ceramic 10 increases, thereby stabilizing coloring.

### <Coloring Liquid for Dental Ceramic>

A coloring liquid for a dental ceramic according to the present embodiment is a coloring liquid for coloring a dental ceramic. As the coloring liquid, for example, a coloring liquid used in the above-described dental ceramic coloring method can be applied.

That is, the coloring liquid for the dental ceramic according to the present embodiment contains at least two types of metal ions in which a difference between standard oxidation-reduction potentials is 0.5 or more and 4.0 or less.

Thus, the coloring liquid for the dental ceramic according to the present embodiment provides the effects the same as those obtained by the above-described dental ceramic coloring method.

That is, as described above, by dipping the one end 10A of the dental ceramic 10 in the coloring liquid containing at least two types of metal ions in which a difference between standard oxidation-reduction potentials is 0.5 or more and 4.0 or less, it is possible to obtain a dental ceramic in which a gradation is formed inside the dental ceramic in a single coloring step using a single coloring liquid.

### EXAMPLES

The present invention will be further described below using experimental examples.

### <Preparation of Test Piece>

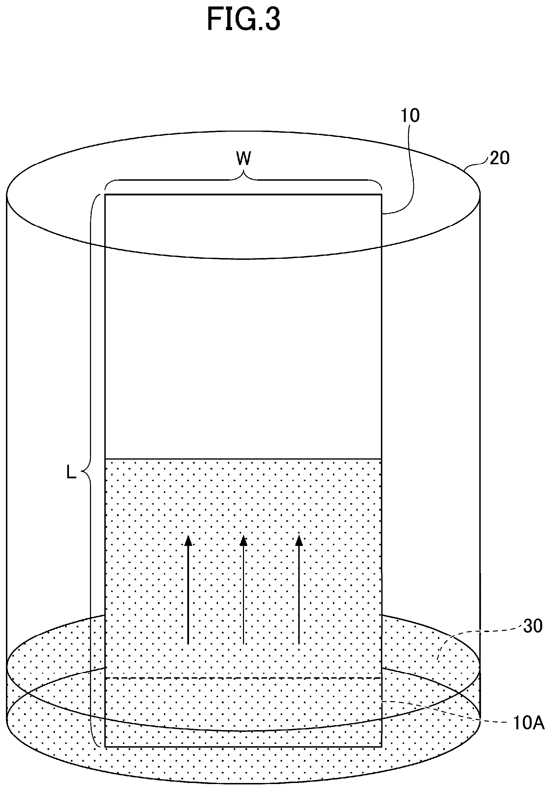

A test piece (dental ceramic) 10 (width **W:** about 18 mm, length L: about 50 mm, and thickness: 1.5 mm) as illustrated in FIG. 3 was provided by cutting a commercially available zirconia calcined body for dental cutting processing (Aadva zirconia disk EI, obtained from GC Corporation) into a plate using a cutting machine.

The cylindrical container 20 (Maruemu Container No. 6) was charged with 2 g of the coloring liquid 30. The test piece 10 was allowed to stand in the container 20 such that the one end 10A (a region of about 5 mm from the bottom end) of the test piece 10 would be dipped in the coloring liquid 30. After being left to stand still for 1 hour to 15 hours, the test piece 10 was taken out from the container 20 and dried at room temperature for 1 hour to 6 hours. The dried test piece 10 was fired at 1,500°C for 2 hours, thereby obtaining a dental ceramic (fired ceramic body).

### <Preparation of Test Piece of Embodiment>

A test piece 10 (width (Y direction): about 15 mm, length (X direction): about 18 mm, and height (Z direction): 13 mm) as illustrated in FIG. 1 was provided by cutting a commercially available zirconia calcined body for dental cutting processing (Aadva zirconia disk EI, obtained from GC Corporation) into a block using a cutting machine.

The periphery 13 of the test piece 10 was covered by a silicone impression material (obtained from SHOFU INC., DUPLICONE (registered trademark)) such that a 1/8 region of the test piece 10 extending from the bottom surface 11 toward the top surface 12 would be exposed. This test piece 10 was set in the container 20 in which the mesh 40 was spread, and the coloring liquid 30 was poured into the container 20, thereby dipping the test piece 10 in the coloring liquid 30 (FIG. 2). After being left to stand still for 18 hours, the test piece 10 was taken out from the container 20 and dried at room temperature for 1 hour to 6 hours, followed by cutting (slicing) to a thickness of about 1.5 mm in width (Y direction). The resulting product was fired at 1,500°C for 2 hours, thereby obtaining a dental ceramic (fired ceramic body).

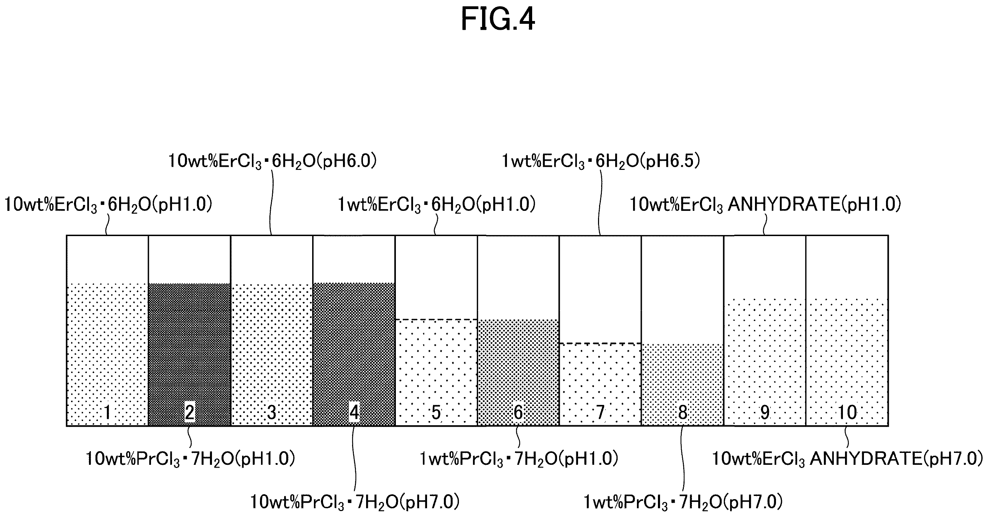

Table 1 and FIGS. 4 to 9 indicate, as the test piece 10 of the embodiment, Experimental Examples 27 and 28 in which the composition of the coloring liquid 30 was different.

**[Table 1]**

| Exptl. Ex. | Composition | pH | Dip time [h] | Shape of test piece |
|---|---|---|---|---|
| 1 | 10wt% ErCl₃·6H₂O | 1.0 | 1.0 | Thin plate |
| 2 | 10wt% PrCl₃·7H₂O | 1.0 | 1.0 | Thin plate |
| 3 | 10wt% ErCl₃·6H₂O | 6.0 | 1.0 | Thin plate |
| 4 | 10wt% PrCl₃·7H₂O | 7.0 | 1.0 | Thin plate |
| 5 | 1wt% ErCl₃·6H₂O | 1.0 | 1.0 | Thin plate |
| 6 | 1wt% PrCl₃·7H₂O | 1.0 | 1.0 | Thin plate |
| 7 | 1wt% ErCl₃·6H₂O | 6.5 | 1.0 | Thin plate |
| 8 | 1wt% PrCl₃·7H₂O | 7.0 | 1.0 | Thin plate |
| 9 | 10wt% ErCl₃ anhydrate | 1.0 | 1.0 | Thin plate |
| 10 | 10wt% ErCl₃ anhydrate | 7.0 | 1.0 | Thin plate |
| 11 | 1wt% FeCl₃·6H₂O | 0.5 | 1.0 | Thin plate |
| 12 | 1wt% FeCl₃·6H₂O | 1.0 | 1.0 | Thin plate |
| 13 | 0.04M FeCl₃·6H₂O | 1.0 | 1.0 | Thin plate |
| 14 | 0.04M Fe(III)-EDTA | 1.0 | 1.0 | Thin plate |
| 15 | 4wt% ErCl₃·6H₂O | 6.0 | 15.0 | Thin plate |
| 16 | 4wt% ErCl₃·6H₃O+EDTA | 6.0 | 15.0 | Thin plate |
| 17 | 1wt%PrCl₃ · 7H₂O | 7.0 | 15.0 | Thin plate |
| 18 | 1wt%PrCl₃ · 7H₂O+EDTA | 7.0 | 15.0 | Thin plate |
| 19 | 4wt% PEG400 | 1.0 | 3.0 | Thin plate |
| | 0.5wt% PrCl₃·7H₂O | | | |
| | 0.25wt% FeCl₃·6H₂O | | | |
| | 1wt% ErCl₃·6H₄O | | | |
| | EDTA | | | |
| 20 | 4wt% PEG400 | 1.0 | 3.0 | Thin plate |
| | 0.5wt% PrCl₃·7H₂O | | | |
| | 1.0wt% FeCl₃·6H₂O | | | |
| | 3.5wt% ErCl₃·6H₄O | | | |
| | EDTA | | | |
| 21 | 2.5wt% PEG400 | 1.0 | 3.0 | Thin plate |
| | 0.3wt% PrCl₃·7H₂O | | | |
| | 0.75wt% FeCl₃·6H₂O | | | |
| | 0.75wt% ErCl₃·6H₄O | | | |
| | EDTA | | | |
| 22 | 2.5wt% PEG400 | 1.0 | 3.0 | Thin plate |
| | 0.3wt% PrCl₃·7H₂O | | | |
| | 1.0wt% FeCl₃·6H₂O | | | |
| | 2.0wt% ErCl₃·6H₄O | | | |
| | EDTA | | | |
| 23 | 4wt% PEG400 | 1.0 | 18.0 | Thin plate |
| | 0.5wt% PrCl₃·7H₂O | | | |
| | 0.25wt% FeCl₃·6H₂O | | | |
| | 1wt% ErCl₃·6H₄O | | | |
| | EDTA | | | |
| 24 | 4wt% PEG400 | 1.0 | 18.0 | Thin plate |
| | 0.5wt% PrCl₃·7H₂O | | | |
| | 1.0wt% FeCl₃·6H₂O | | | |
| | 3.5wt% ErCl₃·6H₄O | | | |
| | EDTA | | | |
| 25 | 2.5wt% PEG400 | 1.0 | 18.0 | Thin plate |
| | 0.3wt% PrCl₃·7H₂O | | | |
| | 0.75wt% FeCl₃·6H₂O | | | |
| | 0.75wt% ErCl₃·6H₄O | | | |
| | EDTA | | | |
| 26 | 2.5wt% PEG400 | 1.0 | 18.0 | Thin plate |
| | 0.3wt% PrCl₃·7H₂O | | | |
| | 1.0wt% FeCl₃·6H₂O | | | |
| | 2.0wt% ErCl₃·6H₄O | | | |
| | EDTA | | | |
| 27 | 4wt% PEG400 | 1.0 | 18.0 | Block |
| | 0.5wt% PrCl₃·7H₂O | | | |
| | 0.25wt% FeCl₃·6H₂O | | | |
| | 1wt% ErCl₃·6H₄O | | | |
| | EDTA | | | |
| 28 | 4wt% PEG400 | 1.0 | 18.0 | Block |
| | 0.5wt% PrCl₃·7H₂O | | | |
| | 1.0wt% FeCl₃·6H₂O | | | |
| | 3.5wt% ErCl₃·6H₄O | | | |
| | EDTA | | | |

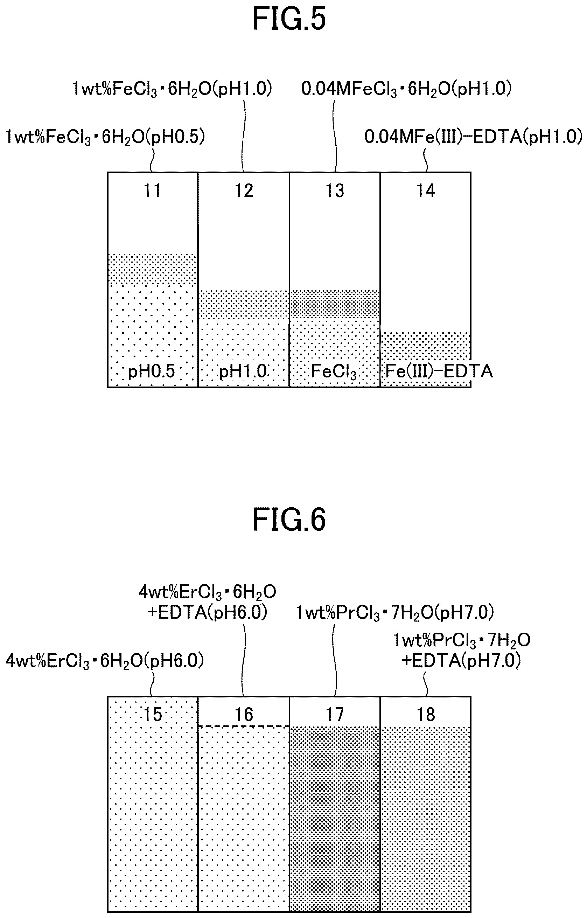

From Table 1, when the concentration of the metal ions was relatively high (the content of the metal ions was 10% by mass), no change in the migration speed depending on the pH could be confirmed (FIG. 4 and Experimental Examples 1 to 4, 9, and 10). On the other hand, when the concentration of the metal ions was relatively low (the content of the metal ions was 1% by mass), it was confirmed that the migration speed in the test piece 10 became higher when the pH was more acidic (FIG. 4 and Experimental Examples 5 to 8, and FIG. 5 and Experimental Examples 11 and 12).

Also, it was confirmed that the migration speed in the test piece 10 tended to be higher when the concentration of the metal ions was relatively high (the content of the metal ions was 10% by mass) than when the concentration of the metal ions was relatively low (the content of the metal ions was 1% by mass) (FIG. 4 and Experimental Examples 1 to 8).

Also, when the metal ion was Er³⁺, it was confirmed that there was little difference in the migration speed in the test piece 10 between the hydrate (ErCl·7H₂O) and the anhydrate (ErCl anhydrate) (Experimental Examples 1, 3, 9, and 10).

Also, when the metal ion was Fe³⁺ or Er³⁺, it was confirmed that the migration speed in the test piece 10 decreased when the coloring liquid contained the chelating agent (EDTA) (FIG. 5 and Experimental Examples 13 and 14, and FIG. 6 and Experimental Examples 15 and 16). On the other hand, when the metal ion was Pr³⁺, no change in the migration speed in the test piece 10 was confirmed even if the chelating agent (EDTA) was contained (FIG. 6 and Experimental Examples 17 and 18).

Also, when comparing Fe³⁺, Er³⁺, and Pr³⁺ metal ions at the same concentration (the content of the metal ions was 1% by mass), it was confirmed that the migration speed in the test piece 10 was higher in Er³⁺ and Pr³⁺ than in Fe³⁺. That is, it was confirmed that the migration speed in the test piece 10 was lower in Fe³⁺ than in Er³⁺ and Pr³⁺ (FIG. 4 and Experimental Examples 5 and 6, and FIG. 5 and Example 12).

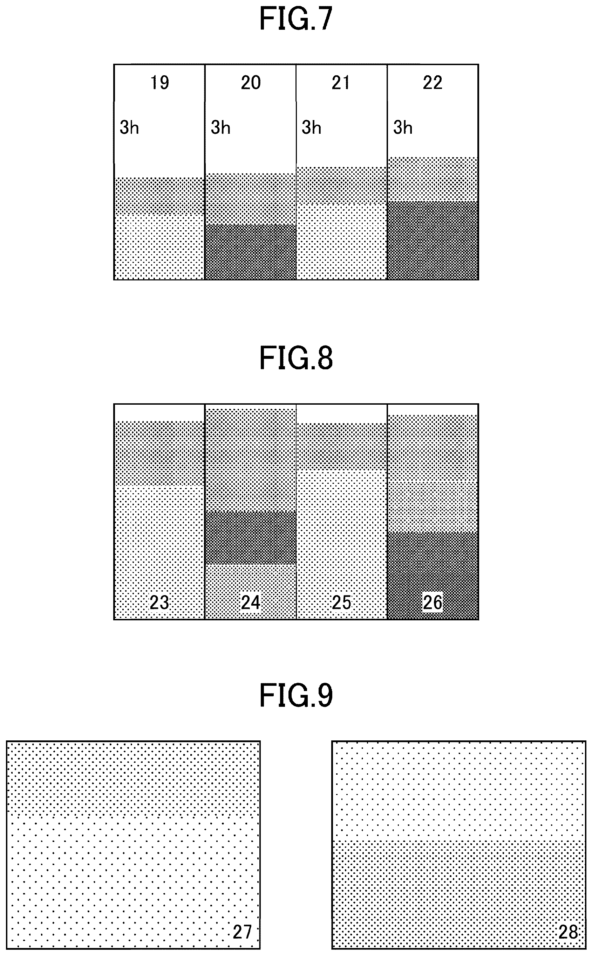

Also, it was confirmed that when the coloring liquid further contained the water-soluble polymer (polyethylene glycol (PEG) 400), unevenness in coloring in the test piece 10 was suppressed (FIG. 7 and Experimental Examples 19 to 22, FIG. 8 and Experimental Examples 23 to 26, and FIG. 9 and Examples 27 and 28).

From these results, it was found that the type of the metal ions, the concentration of the metal ions, the presence or absence of the chelating agent (formation of complexes), the presence or absence of the water-soluble polymer, and the pH of the coloring liquid influenced the migration speed of the metal ions in the test piece 10.

Further, it was confirmed that by selecting the type of the metal ions considering the difference between the standard oxidation-reduction potentials of the metal ions, a gradation was formed inside the dental ceramic (FIG. 7 and Experimental Examples 19 to 22).

As a result, it was found that a sintered ceramic body obtained by dipping the one end 10A of the dental ceramic 10 in the coloring liquid 30, containing at least two types of metal ions in which the difference between the standard oxidation-reduction potentials was 0.5 or more and 4.0 or less, had a gradation inside the dental ceramic in a single coloring step using a single coloring liquid.

Although the embodiments of the present invention have been described above, the present invention is not limited to the specific embodiments. Various modifications and alterations are possible within the scope of the invention as recited in the claims.

Embodiments of the present invention are, for example, as follows.
<1> A method for coloring a dental ceramic, the method including:
   a step of contacting a coloring liquid with the dental ceramic, in which
   the coloring liquid contains at least two types of metal ions in which a difference between standard oxidation-reduction potentials is 0.5 or more and 4.0 or less.
<2> The method for coloring the dental ceramic according to <1> above, in which
   the step of contacting the coloring liquid with the dental ceramic is a step of dipping one end of the dental ceramic in the coloring liquid.
<3> The method for coloring the dental ceramic according to <1> or <2> above, in which
   the standard oxidation-reduction potential of one metal ion of the two types of the metal ions **is** -1.0 V or more and 2.0 V or less.
<4> The method for coloring the dental ceramic according to any one of <1> to <3> above, in which
   the standard oxidation-reduction potential of another metal ion of the two types of the metal ions is -3.0 V or more and -0.7 V or less.
<5> The method for coloring the dental ceramic according to any one of <1> to <4> above, in which
   the coloring liquid further contains a water-soluble polymer.
<6> The method for coloring the dental ceramic according to any one of <1> to <5> above, in which
   the coloring liquid further contains a chelating agent.
<7> The method for coloring the dental ceramic according to any one of <1> to <6> above, in which
   pH of the coloring liquid is 8 or less.
<8> A coloring liquid for coloring a dental ceramic, the coloring liquid including:
   at least two types of metal ions in which a difference between standard oxidation-reduction potentials is 0.5 or more and 4.0 or less.

The present application claims priority to Japanese Patent Application No. 2022-151021, filed on September 22, 2022, the entire contents of which are incorporated herein by reference.

### REFERENCE SIGNS LIST

- 10: Dental ceramic
- 10A: One end
- 11: Bottom surface (bottom end)
- 12: Top surface
- 13: Side surface (periphery)
- 20: Container
- 21: Bottom surface
- 30: Coloring liquid
- 40: Mesh
- 50: Covering material

## Claims

1. A method for coloring a dental ceramic, the method comprising:
a step of contacting a coloring liquid with the dental ceramic, wherein
the coloring liquid contains at least two types of metal ions in which a difference between standard oxidation-reduction potentials is 0.5 or more and 4.0 or less.

2. The method for coloring the dental ceramic according to claim 1, wherein
the step of contacting the coloring liquid with the dental ceramic is a step of dipping one end of the dental ceramic in the coloring liquid.

3. The method for coloring the dental ceramic according to claim 1, wherein
the standard oxidation-reduction potential of one metal ion of the two types of the metal ions is -1.0 V or more and 2.0 V or less.

4. The method for coloring the dental ceramic according to claim 3, wherein
the standard oxidation-reduction potential of another metal ion of the two types of the metal ions is -3.0 V or more and -0.7 V or less.

5. The method for coloring the dental ceramic according to any one of claims 1 to 4, wherein
the coloring liquid further contains a water-soluble polymer.

6. The method for coloring the dental ceramic according to any one of claims 1 to 4, wherein
the coloring liquid further contains a chelating agent.

7. The method for coloring the dental ceramic according to any one of claims 1 to 4, wherein
pH of the coloring liquid is 8 or less.

8. A coloring liquid for coloring a dental ceramic, the coloring liquid comprising:
at least two types of metal ions in which a difference between standard oxidation-reduction potentials is 0.5 or more and 4.0 or less.
